# EUROPÄISCHE PATENTSCHRIFT

(11) **EP 1 642 538 B1**
(45) Veröffentlichungstag und Bekanntmachung des Hinweises auf die Patenterteilung: **07.11.2007**
(21) Anmeldenummer: 05021336.2
(22) Anmeldetag: 29.09.2005
(51) Int. Cl.: A61B 17/17

(54) **Vorrichtung zum Führen eines Bohrwerkzeuges**
Device for guiding a drilling tool
Dispositif pour guider un outil de forage

(30) Priorität: 29.09.2004 DE 102004048042
(43) Veröffentlichungstag der Anmeldung: 05.04.2006
(73) Patentinhaber: Karl Storz GmbH & Co. KG, 78532 Tuttlingen (DE)
(72) Erfinder: Weiler, Andreas, Dr., 13505 Berlin (DE); Berberich, Sascha, 78532 Tuttlingen (DE)
(74) Vertreter: Weller, Wolfgang

(56) Entgegenhaltungen:
- EP-A- 1 178 356
- US-A- 4 722 331
- US-A- 5 152 764
- US-A- 5 688 284
- US-A- 5 891 150
- US-A1- 2001 053 934
- US-A1- 2002 087 160
- US-A1- 2004 087 953

## Beschreibung

Die Erfindung betrifft eine Vorrichtung zum Führen eines Bohrwerkzeuges zum Einbringen einer Bohrung in einem Knochen, die eine bereits vorhandene erste Bohrung schneidet, mit einem ersten stabförmigen Körper, dessen erstes Ende in die bereits vorhandene erste Bohrung im Knochen einführbar ist, dessen zweites Ende mit einem Arm versehen ist, der eine Führung für das Bohrwerkzeug trägt, dessen Längsachse den stabförmigen Körper im Bereich dessen ersten Endes schneidet, wobei der Arm einen kreisbogenförmigen Abschnitt aufweist, wobei der Mittelpunkt des kreisbogenförmigen Abschnitts im Schnittpunkt der Längsachse des Bohrwerkzeuges mit dem stabförmigen Körper liegt, und wobei im Bereich des Schnittpunkts im stabförmigen Körper eine durch diesen hindurchgehende Öffnung ausgespart ist, über die das Bohrwerkzeug durch den stabförmigen Körper hindurch führbar ist.

Eine derartige Vorrichtung findet Einsatz beim Fixieren eines in einen Knochen eingesetzten Sehnenimplantates, meist im Bereich des Kniegelenks.

Dazu wird mit einem Bohrwerkzeug eine erste Bohrung in den Knochen eingebracht, beispielsweise in den Oberschenkelknochen, die als Sacklochbohrung ausgestaltet ist. In diese Sacklochbohrung wird das Sehnenimplantat eingeschoben.

Zur Fixierung dieses Sehnenimplantats wird ein Querbolzen eingebracht, der quer in die Sehne oder in eine Schlaufe in der Sehne eingetrieben wird, so dass die Sehne durch diesen Querbolzen, auch Cross-Pin genannt, gegen Abziehen aus der ersten Bohrung fixiert ist.

Zum Einbringen des Cross-Pins ist es notwendig, eine weitere zweite Bohrung einzubringen, die so ausgerichtet ist, dass diese die erste Bohrung trifft, also quer, meist im rechten Winkel zu dieser, verläuft.

Da aus dieser Querrichtung die Lage der Sacklochbohrung im Knochen für den Operateur nicht ersichtlich ist, wurden Hilfs- oder Zielgeräte geschaffen, um diese zweite Querbohrung zum Einbringen des Cross-Pins genau lage- bzw. zielgerecht anzubringen, d.h. derart, dass diese zweite Bohrung die erste Bohrung trifft.

Eine solche Vorrichtung ist aus der US 5,891,150 bekannt.

Die Vorrichtung kann zum Befestigen eines Bandes am Knochen verwendet werden. Die Vorrichtung weist ein etwa kreisbogenförmiges Element auf, an dessen einem Ende ein stabförmiges Element angeordnet ist, das in eine bereits vorhandene erste Bohrung im Knochen einführbar ist. Am distalen Ende des stabförmigen Elements ist eine als Schlitz ausgebildete Öffnung vorhanden. Am zweiten Ende des etwa kreisbogenförmigen Elements ist eine Öffnung zur Aufnahme eines Bohrwerkzeugs vorhanden. Das Bohrwerkzeug ist durch diese Öffnung derart geführt, dass dessen Spitze durch die als Schlitz ausgebildete Öffnung des stabförmigen Elements durchgeht. Dadurch wird mit dem Bohrwerkzeug eine zweite Bohrung in dem Knochen bewerkstelligt, die sich quer zu der ersten Bohrung erstreckt.

In der US 2001/0053934 A1 ist ein Instrument offenbart, das zur Rekonstruktion eines Kreuzbands dient. Das Instrument weist ein kreisbogenförmiges Element auf, auf dem ein stabförmiges Element beweglich aufgenommen ist. Ein distales Ende des stabförmigen Elements ist mit einer als Schlitz ausgebildeten Öffnung versehen. An einem Ende des kreisbogenförmigen Elements ist eine Führung für ein Bohrwerkzeug angeordnet. Bei der Rekonstruktion des Kreuzbands wird zuerst das stabförmige Element in eine bereits schon im Knochen vorhandene Öffnung eingeführt und mit dem in der Führung aufgenommenen Bohrwerkzeug wird eine zweite sich quer zu der ersten Bohrung erstreckende Bohrung im Knochen bewerkstelligt.

Aus der US 2004/0087953 A1 ist eine Vorrichtung bekannt, die einen L-förmigen Körper aufweist, an dessen einem Ende ein stabförmiges Element angeordnet ist. Am zweiten Ende ist dagegen eine Führung für ein Bohrwerkzeug angeordnet. Bei einer chirurgischen Operation wird das stabförmige Element mit einer an dessen distalem Ende angeordneten Öffnung in eine erste Bohrung im Knochen eingeführt und mit dem Bohrwerkzeug, das durch die Führung geführt wird, wird eine zweite sich quer zu der ersten Bohrung erstreckende Bohrung für einen einzusetzenden Cross-Pin im Knochen beschaffen. Beim Bewerkstelligen der zweiten Bohrung geht die Spitze des Bohrwerkzeugs durch die in dem stabförmigen Körper vorhandenen Öffnung hindurch.

In der US 4,722,331 ist eine Vorrichtung bekannt gegeben, die einen kreisbogenförmigen Körper aufweist, der aus zwei Teilen besteht, die mit einer Schraube verbunden sind und um eine Achse der Schraube schwenkbar sind. An einem Ende eines Teiles des kreisbogenförmigen Körpers ist eine Führung für ein Bohrwerkzeug angeordnet. Am zweiten Ende des Körpers ist ein stabförmiges Element angeordnet. Dieses Element wird von einem Chirurg mit Hilfe eines Arthoskops in einem Gelenk, z.B. Kniegelenk, positioniert. Dann werden die zwei Teile des kreisbogenförmigen Körpers in eine gewünschte Position gebracht und in diese durch Verwendung der Schraube fixiert. Mit dem Bohrwerkzeug, das in Bezug auf das stabförmige Element am gegenseitigen Ende des kreisbogenförmigen Körpers angeordnet ist, wird eine Bohrung im Knochen bewerkstelligt.

Aus der US 2002/0087160 A1 ist eine Vorrichtung bekannt, die zum Fixieren eines Implantats bspw. im Kniegelenk dient. Diese Vorrichtung weist ein Element zum Führen eines Bohrwerkzeugs auf. In diesem Element ist eine hindurchgehende Öffnung vorgesehen, die sich an einer Seite ausweitet. Die Ausweitung der Öffnung dient zur Einlaufführung des Bohrwerkzeugs.

In der US 5,152,764 ist eine Vorrichtung offenbart, die zur Rekonstruktion des Kreuzbands dient. Die Vorrichtung weist einen U-förmigen Körper auf, dessen einer Schenkel einen stabförmigen Körper bildet. Am distalen Ende des Schenkels, der in eine Bohrung im Knochen einführbar ist, ist eine Öffnung angeordnet. An dem distalen Ende des sich außerhalb des Körpers befindlichen Schenkels ist ebenfalls eine Öffnung angeordnet, die zur Führung eines Bohrwerkzeugs dient. Mit dieser Vorrichtung wird eine zweite Bohrung bewerkstelligt, die sich quer zu der ersten Bohrung erstreckt, indem das Bohrwerkzeug durch die Öffnung des sich in den Knochen befindlichen hindurchgeführt wird.

Bei der US 5,688,284 ist ein stabförmiger Körper vorgesehen, der in die bereits vorhandene erste Bohrung eingeschoben werden kann. An seinem zweiten außerhalb des Knochens liegendes Endes ist der stabförmige Körper mit einem seitlich vorspringenden Arm versehen, der ein äußeres Ende aufweist.

Das äußere Ende des Armes ist als kreisbogenförmiger Abschnitt ausgebildet. Auf dem kreisbogenförmigen Abschnitt ist verschieblich die Führung für das zweite Bohrwerkzeug aufgenommen. Der kreisbogenförmige Abschnitt hat einen Mittelpunkt, der im Schnittpunkt der Längsachse des Bohrwerkzeuges mit dem stabförmigen Körper liegt.

Somit trifft die Spitze des zweiten Bohrwerkzeugs immer exakt auf dieselbe Stelle des ersten stabförmigen Körpers, unabhängig davon, in welcher Stellung sich die Führung für das Bohrwerkzeug längs des kreisbogenförmigen Abschnittes befindet.

Nachteilig an einer Vorrichtung der eingangs genannten Art ist, dass das Bohrwerkzeug von einer Seite nur bis an den ersten stabförmigen Körper herangeführt werden kann.

Es ist daher Aufgabe der vorliegenden Erfindung, hier Abhilfe zu schaffen und eine Vorrichtung der eingangs genannten Art derart weiterzubilden, dass der einzusetzende Cross-Pin lage- bzw. zielgerecht eingesetzt werden kann.

Erfindungsgemäß wird die Aufgabe dadurch gelöst, dass die Öffnung als durchgehende Bohrung ausgebildet ist, durch die das Bohrwerkzeug in unterschiedlichen Winkelstellungen führbar ist, und dass die Öffnung Anschrägungen aufweist, die als Einlaufführung für eine Spitze des Bohrwerkzeuges dienen, und dass entlang des kreisbogenförmigen Abschnitts die Führung für das Bohrwerkzeug bewegbar ist.

Diese Maßnahme hat den erheblichen Vorteil, dass die Querbohrung durch den stabförmigen Körper hindurchgehend auch auf der gegenüberliegenden Seite weiterreichend eingebracht werden kann. Für einen Cross-Pin ist es notwendig, dass dieser sich beidseits in Querrichtung zur ersten Bohrung erstreckt, damit dieser auf beiden Seiten im Knochen verankert ist. Dazu ist es notwendig, entsprechend die Querbohrung in Bohrrichtung auch auf der gegenüberliegenden Seite zur ersten Bohrung fortzuführen. Dieser Abschnitt kann ggf. mit einem geringeren Durchmesser versehen werden, in den der Cross-Pin eingetrieben wird.

Die nunmehr vorgeschlagene Konstruktion des Vorsehens einer Öffnung in dem stabförmigen Körper erlaubt das Bewerkstelligen einer Querbohrung, die über die erste Bohrung hinaus reicht, in einem einzigen Arbeitsgang.

Die Ausbildung der Öffnung als durchgehende Bohrung, durch die das Bohrwerkzeug in den unterschiedlichen Winkelstellungen führbar ist, ermöglicht, dass in allen möglichen Winkelstellungen das Bohrwerkzeug durch die Öffnung in dem stabförmigen Körper hindurchreichen kann.

Ferner ermöglicht das Versehen der Öffnung mit Anschrägungen, die als Einlaufführung für die Spitze des Bohrwerkzeugs dienen, dass das Bohrwerkzeug auf den Zielpunkt gerichtet geführt wird.

Sollten beim Eintreiben des zweiten Bohrwerkzeuges geringe Abweichungen oder Ablenkungen auf Grund anatomischer Gegebenheiten erfolgen, führen die Anschrägungen die Spitze des Bohrwerkzeuges zielgerichtet in die Öffnung ein, durch diese hindurch, so dass dann auch zielgerecht die Bohrung auf der gegenüberliegenden Seite weitergeführt werden kann.

In einer weiteren Ausgestaltung der Erfindung ist der kreisförmige Abschnitt bewegbar am Arm angebracht.

Diese Maßnahme erhöht die Flexibilität dahingehend weiter, dass die Führung nicht nur entlang des kreisbogenförmigen Abschnitts bewegt werden kann, sondern dass dieser kreisförmige Abschnitt selbst noch zusätzlich bewegt werden kann.

In einer Ausgestaltung ist dazu vorgesehen, dass der kreisbogenförmige Abschnitt schwenkbar angebracht ist.

Durch diese Schwenkbarkeit kann quasi eine gesamte Kugelabschnittsfläche als Zielfläche ausgewählt werden, die durch das Ausmaß des kreisförmigen Abschnittes abdeckbar ist.

In einer weiteren Ausgestaltung ist der kreisbogenförmige Abschnitt verschiebbar angebracht.

Diese Maßnahme erhöht die Flexibilität dahingehend weiter, dass der kreisbogenförmige Abschnitt längs seiner Kreisbogenlinie noch zusätzlich hin und her verschoben werden kann.

Wird diese Maßnahme in Zusammenhang mit der zuvor erwähnten Maßnahme der Schwenkbarkeit angewendet, kann mit einem relativ kurzen kreisförmigen Abschnitt ein sehr großer Kugeloberflächenabschnitt abgedeckt werden, so dass quasi der gesamte überhaupt zur Verfügung stehende Bereich insbesondere im Bereich des Oberschenkel- (Femur) oder des Unterschenkelknochens (Tibia) angezielt werden kann.

Für den Fall, dass ein zweiter Cross-Pin angebracht werden soll oder der Schnittpunkt der Achse des Bohrwerkzeuges mit der ersten Bohrung in der Längslage verändert werden soll, kann der kreisbogenförmige Abschnitt als solcher längs des Armes verschoben werden.

In einer weiteren Ausgestaltung der Erfindung weist der kreisförmige Abschnitt eine Winkelskala auf.

Dadurch ist es möglich, die exakte Winkelstellung des Kanals zum Einschieben des Cross-Pins zur Längsachse der ersten Bohrung festzustellen oder zu dokumentieren.

In einer weiteren Ausgestaltung der Erfindung ist der Arm als U-förmiger Bügel ausgebildet, dessen einer Schenkel den stabförmigen Körper bildet.

Diese Maßnahme hat den Vorteil, dass eine sehr stabile schlank bauende und auch einfach herzustellende Ausgestaltung des Grundkörpers möglich ist, dessen einer Schenkel mit seinem ersten Ende in die bereits vorhandene erste Bohrung zum Einbringen des Sehnenimplantats eingeschoben werden kann. Anschließend kann der Operateur die gewünschten Manipulationen zum Ansetzen, Ausrichten und Zielen des zweiten Bohrwerkzeuges durchführen.

In einer weiteren Ausgestaltung der Erfindung ist der kreisförmige Abschnitt am Ende des dem ersten stabförmigen Körper gegenüberliegenden Ende des U-förmigen Körpers angebracht.

Diese Maßnahme hat den Vorteil, dass der U-förmige Körper für den Operateur gut handhabbar ist und dass das äußere zweite Ende des U auf Höhe des ersten in die erste Bohrung eingeschobenen inneren Endes des U liegt. In diesem Bereich des "U" sind ja die im Kniegelenk vorhandenen Erweiterungen des Ober- bzw. Unterschenkelknochens vorhanden, die von dem kreisbogenförmigen Abschnitt, der die Führung trägt, umspannt werden.

Die Erfindung wird nachfolgend an Hand einiger ausgewählter Ausführungsbeispiele in Zusammenhang mit den beiliegenden Zeichnungen näher beschrieben und erläutert. Es zeigen:
- Fig. 1: stark schematisiert eine Seitenansicht eines ersten Ausführungsbeispiels einer erfindungsgemäßen Vorrichtung,
- Fig. 2: eine der Fig. 1 vergleichbare Seitenstellung in einer anderen Einstellung bzw. Ausgestaltung des kreisbogenförmigen Abschnitts,
- Fig. 3: perspektivisch und stark schematisiert die erfindungsgemäße Vorrichtung von Fig. 1 nach Bewerkstelligen der Querbohrung für den Cross-Pin durch das Bohrwerkzeug,
- Fig. 4: eine ausschnittsweise stark vergrößerte Ansicht des ersten Endes des stabförmigen Körpers im Bereich der Öffnung zum Durchführen des Bohrwerkzeuges, wobei verschiedene Winkelstellungen des Bohrwerkzeugs dargestellt sind,
- Fig. 5: eine der Darstellung von Fig. 3 vergleichbare Darstellung mit einem weiteren Ausführungsbeispiel einer erfindungsgemäßen Vorrichtung, und
- Fig. 6: eine Anwendung der in Fig. 5 dargestellten Vorrichtung in einer anderen Winkelstellung des Bohrwerkzeuges und bei einer anderen Operationstechnik.

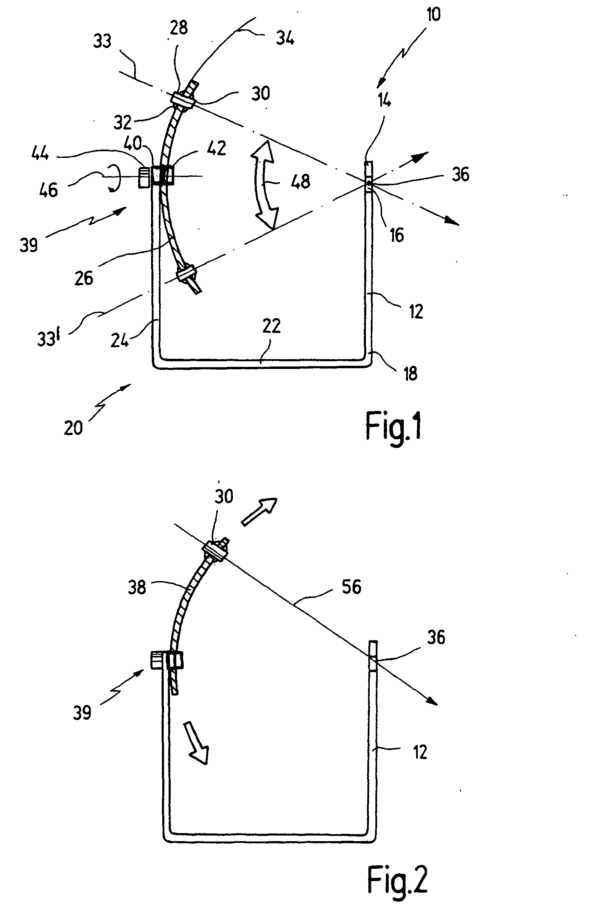

Ein in den Fig. 1 bis 4 dargestelltes erstes Ausführungsbeispiel einer erfindungsgemäßen Vorrichtung ist in ihrer Gesamtheit mit der Bezugsziffer 10 versehen.

Die Vorrichtung 10 weist einen lang erstreckten stabförmigen Körper 12 mit kreisförmigem Querschnitt auf.

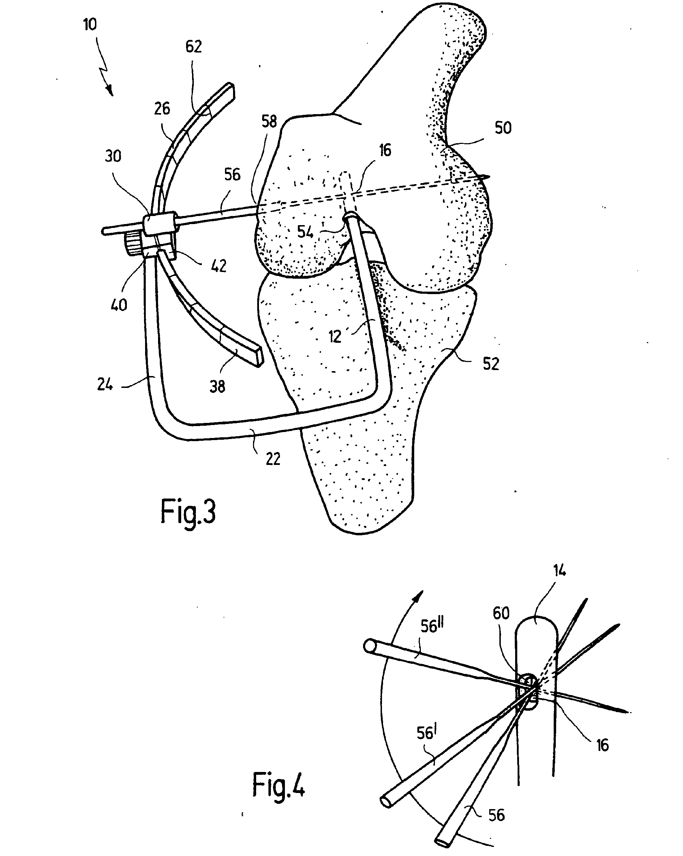

Im Bereich dessen erstes Endes 14 ist eine Öffnung 16 in Form einer durchgehenden Bohrung, wie das später noch näher in Zusammenhang mit Fig. 4 beschrieben wird, vorhanden.

Das dem ersten Ende 14 gegenüberliegende zweite Ende 18 setzt sich in einem etwa rechtwinklig vorspringenden abgewinkelten Arm 20 fort.

Der Arm 20 besteht aus einem Querstab 22 und einem hochstehenden Schenkel 24. Wie aus Fig. 1 ersichtlich, ist der Arm 20 so ausgebildet, dass in Zusammenhang mit dem stabförmigen Körper 12 ein etwa U-förmiger gleichschenkliger Körper entsteht, der aus ein und demselben stabförmigen Material durch entsprechende Biegevorgänge hergestellt werden kann.

Am äußeren Endbereich des hochstehenden Schenkels 24 ist ein kreisbogenförmiger Abschnitt 26 angebracht. Der kreisbogenförmige Abschnitt 26 trägt eine Führung 28 für ein Bohrwerkzeug.

Die Führung 28 ist als Führungshülse 30 ausgebildet, die längs des kreisförmigen Abschnitts 26 hin und her verschoben werden kann. Eine Feststellschraube 32 dient dazu, diesen Verschiebevorgang zu ermöglichen und die Führungshülse 30 in einer bestimmten Position zu fixieren. Die Führungshülse 30 ist so ausgerichtet, dass deren Mittellängsachse 33 in Richtung eines Punktes 36 ausgerichtet ist, der in etwa in der Mitte der Öffnung 16 im stabförmigen Körper 12 liegt. Dieser Punkt stellt den Mittelpunkt 36 des kreisbogenförmigen Abschnitts 26 dar. Wird also in die Führungshülse 30, wie das später noch näher beschrieben wird, ein Bohrwerkzeug eingeschoben, wird dies längs dessen Längsachse 33 zielgerichtet auf die Öffnung 16 im ersten Ende 14 des stabförmigen Körpers 12 ausgerichtet, wie das durch die doppelstrichpunktierte Linie angedeutet ist.

Durch Lösen der Feststellschraube 32 kann die Führungshülse 30 beispielsweise in der Darstellung von Fig. 1 nach unten bewegt werden, bis die Ausrichtung des Bohrwerkzeuges der durch die strichpunktierte Längsachse 33' angedeutete Ausrichtung entspricht.

In all diesen unterschiedlichen Winkelstellungen entlang des kreisbogenförmigen Abschnitts 26 ist die Mittellängsachse des Bohrwerkzeuges exakt auf die Öffnung 16 ausgerichtet.

Es entsteht also hier in dieser Ebene der durch den Doppelpfeil 48 angezeigte Schwenkbereich für das Bohrwerkzeug.

Der kreisbogenförmige Abschnitt 26 ist über eine Arretierung 39 schwenkbar am Schenkel 24 angebracht.

Die Arretierung 39 weist zwei Klemmbacken 40 und 42 auf, die über eine Klemmschraube 44 gelöst oder festgestellt werden können. Bei gelöster Arretierung 39 kann der kreisbogenförmige Abschnitt 46 um eine Achse 46 aus der Zeichenebene von Fig. 1 heraus verschwenkt werden und in einer entsprechenden gewünschten Winkelstellung arretiert werden. Der Freiheitsgrad der Ausrichtungen der Längsachsen 33, 33' des Bohrwerkzeuges, das in die Führungshülse 30 eingeschoben wird, liegt somit innerhalb eines Kegelkörpers.

Falls ein anderer Punkt am stabförmigen Körper 12 angezielt werden möchte, kann der kreisbogenförmige Abschnitt 26 längs des Schenkels 24 verschoben werden. Es können dann weitere Öffnungen im stabförmigen Körper 12 vorhanden sein, oder in diesem ist eine längsverlaufende Langlochöffnung vorhanden, so dass das Bohrwerkzeug durch den Körper 12 hindurchtreten kann.

In Fig. 2 ist dargestellt, dass durch Lösen der Arretierung 39 der kreisbogenförmige Abschnitt nicht nur um die Achse 46 gedreht, sondern auch in Richtung des Kreises 34 verschoben bzw. bewegt werden kann. Durch das entsprechende Verschieben, wie das beispielsweise in Fig. 2 dargestellt ist, ist es möglich, den Freiheitsgrad in seinem Ausmaß noch weiter zu erweitern, so dass ein wesentlich größerer Kegelwinkelbereich abgedeckt werden kann.

An Hand der Fig. 3 und 4 soll nunmehr im praktischen Einsatz erläutert werden, was der Sinn und Zweck und der Vorteil dieser flexiblen Bewegung der Führungshülse 30 und des kreisbogenförmigen Abschnitts 26 ist.

In Fig. 3 ist stark schematisiert ein Kniegelenk dargestellt, und zwar Femur 50 (Oberschenkelknochen) und Tibia 52 (Unterschenkelknochen). Am Oberschenkelknochen 50 soll eine Sehne verankert werden, die als Ersatz für ein defektes Kreuzband dienen soll.

Dazu wurde in den Femur 50 eine erste Bohrung 54 in Form einer Sacklochbohrung eingebracht. Diese kann durch eine durchmessergeringe Bohrung bis zur Außenseite fortgesetzt sein, um beispielsweise Fäden zur Halterung oder zum Einziehen der Sehne durchzuführen. Zum zielgerechten Einbringen dieser ersten Bohrung 54 kann beispielsweise ein Zielgerät eingesetzt werden, wie es aus der US 5,350,383 bekannt ist.

In Fig. 3 ist dargestellt, wie in diese erste Bohrung 54 der Endbereich des stabförmigen Körpers 12 der Vorrichtung 10 eingeschoben ist. Dazu ist der Außendurchmesser des stabförmigen Körpers 12 so gewählt, dass er in die erste Bohrung 54 eingeschoben werden kann.

Wie aus Fig. 3 ersichtlich, liegt die Öffnung 16 im stabförmigen Körper 12 innerhalb der ersten Bohrung 54.

Der kreisbogenförmige Abschnitt 26 liegt in einer Ebene, die durch den U-förmigen Körper, zusammengesetzt aus dem stabförmigen Körper 12 und dem Arm 20, definiert ist. Die Führungshülse 30 liegt auf Höhe der Arretierung 39 und es wurde durch die Öffnung der Führungshülse 30 ein Bohrwerkzeug 56 eingeschoben und so weit in den Oberschenkelknochen 50 eingetrieben, dass eine zweite Bohrung 58 entsteht, die quer zur ersten Bohrung 54 verläuft, diese schneidet und sich über die erste Bohrung 54 hinaus erstreckt. Im dargestellten Ausführungsbeispiel ist das Bohrwerkzeug 56 durch den ganzen Oberschenkelknochen 50 hindurchgetrieben. Durch die zuvor erwähnte Ausrichtung wurde das Bohrwerkzeug 56 exakt durch die Öffnung 16 im stabförmigen Körper 12 hindurchgeführt, um die zweite Bohrung 58 anzubringen.

Nach Abziehen des Bohrwerkzeuges 56 wird in die zweite Bohrung 58 ein sog. Cross-Pin eingetrieben, der dazu dient, eine zuvor in die erste Bohrung 54 eingebrachte Sehne zu verankern bzw. zu fixieren.

Aus der vergrößerten ausschnittsweisen Darstellung von Fig. 4 ist ersichtlich, dass die Öffnung 16 Anschrägungen 60 aufweist.

Dadurch wird in jeder Winkellage das Bohrwerkzeug 56 in die Öffnung 16 sanft geleitet bzw. geführt.

Je nach Ausmaß der Länge des kreisförmigen Abschnitts 26 ist die Öffnung als Langlochöffnung mit entsprechend umlaufender Anschrägung 60 ausgestaltet. Es ist möglich, geringe Fehlabweichungen beim Eintreiben des Bohrwerkzeuges 56 dadurch zu korrigieren, dass dieses durch die Anschrägungen 60 zielgerecht in die Öffnung 16 eingeführt wird.

Aus Fig. 3 ist ersichtlich, dass am kreisförmigen Abschnitt 26 eine Winkelskala 62 angebracht ist, um die Verschiebung der Führung 30 exakt einstellen oder dokumentieren zu können. Man kann beispielsweise die in Fig. 3 dargestellte Position als 0°-Position definieren und Abweichungen nach oben als positive und Abweichungen nach unten als negative Abweichungsgrade definieren.

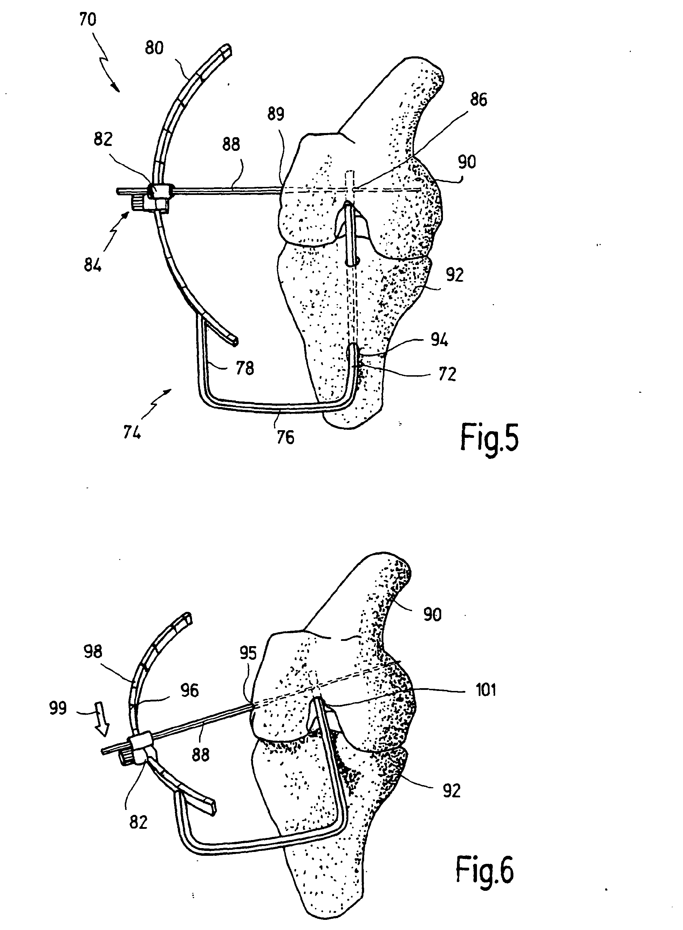

Bei einem in den Fig. 5 und 6 dargestellten weiteren Ausführungsbeispiel einer erfindungsgemäßen Vorrichtung 70 ist ebenfalls ein erster stabförmiger Körper 72 vorgesehen, der mit einem Arm 74 verbunden ist. Auch hier weist der Arm 74 einen Querstab 76 auf, der in einen hochstehenden Schenkel 78 übergeht, der etwa parallel zum stabförmigen Körper 72 verläuft. Am äußeren Ende des Schenkels 78 ist der kreisbogenförmige Abschnitt 80 fest angebracht, beispielsweise angeschweißt. Der Schenkel 78 ist wesentlich kürzer als der stabförmige Körper 72.

Auf dem kreisbogenförmigen Abschnitt 80 ist wieder verschiebbar eine Führung 82 für ein Bohrwerkzeug 88 aufgenommen. Über eine Arretierung 84 kann die Führung 82 längs des kreisbogenförmigen Abschnitts 80 verschoben und fixiert werden. Auch hier ist wieder die Ausrichtung der Führung 82 so, dass ein durch sie hindurchgeschobenes Bohrwerkzeug 88 exakt auf eine Öffnung 86 im stabförmigen Körper 72 ausgerichtet ist, wie das zuvor beschrieben wurde.

In Fig. 5 ist dargestellt, dass der stabförmige Körper 72 in eine erste Bohrung 94 eingeschoben ist, die sich sowohl durch den Unterschenkelknochen 92 (Tibia) als auch in den Oberschenkelknochen 90 (Femur) hinein erstreckt.

In Fig. 6 ist der Einsatz der Vorrichtung 70 bei einer etwas anderen Operationstechnik dargestellt, die ähnlich zu der in Fig. 3 beschriebenen ist, hier ist also die erste Bohrung 101 lediglich in den Oberschenkelknochen 90 eingetrieben. Die Führung 82 ist dabei längs des kreisförmigen Abschnitts 80 gegenüber der Darstellung von Fig. 5 nach unten verschoben, beispielsweise um -20°, wie das durch den Pfeil 99 angedeutet ist. Auch hier erlaubt eine entsprechende Skala 96, dies lagerecht auszuführen. Der kreisbogenförmige Abschnitt 80 ist, wie ersichtlich, als eine Schiene 98 mit rechteckförmigem Querschnitt ausgebildet. Durch Lösen der Arretierung bzw. deren Stellschraube werden die Klemmbacken, zwischen denen die Schiene 98 aufgenommen ist, etwas geöffnet, so dass dann die Führung 82 entsprechend verschoben werden kann. Ist die gewünschte Position erreicht, wird die Arretierung festgestellt und das Bohrwerkzeug 88 kann eingeschoben werden.

Dies zeigt, dass mit einer erfindungsgemäßen Vorrichtung sehr flexibel auf die anatomischen Gegebenheiten eingegangen werden kann, beispielsweise wenn im Bereich des Oberschenkelknochens entsprechende Fehlstellen oder Lücken vorhanden sein sollten, die kein sinnvolles Ansetzen des Bohrwerkzeuges ermöglichen.

Wie zuvor beschrieben, wird nach Einbringen der zweiten Bohrung 95 dann ein entsprechender Cross-Pin eingetrieben, um die zwischenzeitlich in die erste Bohrung 94 eingebrachte Sehne bzw. den Sehnenersatz zu fixieren.

## Patentansprüche

1. Vorrichtung zum Führen eines Bohrwerkzeuges (56, 88) zum Einbringen einer Bohrung (58, 89, 95) in einem Knochen, die eine bereits vorhandene erste Bohrung (54, 94, 101) schneidet, mit einem ersten stabförmigen Körper (12, 72), dessen erstes Ende (14) in die bereits vorhandene erste Bohrung (54, 94, 101) im Knochen einführbar ist, dessen zweites Ende (18) mit einem Arm (20, 74) versehen ist, der eine Führung (28, 82) für das Bohrwerkzeug (56, 88) trägt, dessen Längsachse (33, 33') den stabförmigen Körper (12, 72) im Bereich dessen ersten Endes (14) schneidet, wobei der Arm (20, 74) einen kreisbogenförmigen Abschnitt (26, 80) aufweist, wobei der Mittelpunkt (36) des kreisbogenförmigen Abschnittes (26, 80) im Schnittpunkt der Längsachse (33, 33') des Bohrwerkzeugs (56, 88) mit dem stabförmigen Körper (12, 72) liegt, wobei im Bereich des Schnittpunkts im stabförmigen Körper (12, 72) eine durch diesen hindurchgehende Öffnung (16, 86) ausgespart ist, über die das Bohrwerkzeug (56, 56', 56") durch den stabförmigen Körper (12, 72) hindurch führbar ist, wobei die Öffnung (16, 86) als durchgehende Bohrung ausgebildet ist, durch die das Bohrwerkzeug (56, 56', 56'') in unterschiedlichen Winkelstellungen führbar ist, die Öffnung (16) Anschrägungen (60) aufweist, die als Einlaufführung für eine Spitze des Bohrwerkzeuges (56, 56', 56") dienen, und die Führung (28, 82) für das Bohrwerkzeug (56, 58) entlang dem kreisbogenförmigen Abschnitt (26, 80) bewegbar ist.

2. Vorrichtung nach Anspruch 1, **dadurch gekennzeichnet, dass** der kreisbogenförmige Abschnitt (26) bewegbar am Arm (20) angebracht ist.

3. Vorrichtung nach Anspruch 2, **dadurch gekennzeichnet, dass** der kreisbogenförmige Abschnitt (26) schwenkbar am Arm (20) angebracht ist.

4. Vorrichtung nach Anspruch 2 oder 3, **dadurch gekennzeichnet, dass** der kreisbogenförmige Abschnitt (26) verschiebbar am Arm (20) angebracht ist.

5. Vorrichtung nach einem der Ansprüche 1 bis 4, **dadurch gekennzeichnet, dass** der kreisbogenförmige Abschnitt (26, 80) eine Winkelskala (62, 96) aufweist.

6. Vorrichtung nach einem der Ansprüche 1 bis 5, **dadurch gekennzeichnet, dass** der Arm (20, 74) als U-förmiger Bügel ausgebildet ist, dessen einer Schenkel den stabförmigen Körper (12, 72) bildet.

7. Vorrichtung nach Anspruch 6, **dadurch gekennzeichnet, dass** der kreisbogenförmige Abschnitt (26) dem ersten Ende (14) des stabförmigen Körpers (12) gegenüberliegend am U-förmigen Körper angebracht ist.

## Claims

1. Device for guiding a drilling tool (56, 88) for introducing a bore (58, 89, 95) into a bone, intersecting an already existing first bore (54, 94, 101), having a first rod-shaped body (12, 72), the first end (14) of which can be introduced into the already existing first bore (54, 94, 101) in the bone, the second end (18) of which is provided with an arm (20, 74) which bears a guide (28, 82) for the drilling tool (56, 88) and the longitudinal axis (33, 33') of which intersects the rod-shaped body (12, 72) in the region of its first end (14), said arm (20, 24) has an arcuate portion (26, 80), a center point (36) of the arcuate portion (26, 80) lies in a point of intersection of the longitudinal axis (33, 33') of the drilling tool (56, 88) with the rod-shaped body (12, 72), a continuous opening (16, 18) is cut out in the rod-shaped body (12, 72) in the region of the point of intersection, through which the drilling tool (56, 56', 56") can be guided through the rod-shaped body (12, 72), wherein the opening (16, 86) is formed as a continuous bore through which the drilling tool (56, 56', 56") can be guided in various angular positions, said opening (16) has bevels (60) which serve as a running-in guide for a tip of the drilling tool (56, 56', 56"), and wherein said guide (28, 82) for the drilling tool (56, 58) can be moved along the arcuate portion (26, 28).

2. Device of claim 1, **characterized in that** the arcuate portion (26) is movably attached to the arm (20).

3. Device of claim 2, **characterized in that** the arcuate portion (26) is pivotably attached to the arm (20).

4. Device of claims 2 or 3, **characterized in that** the arcuate portion (26) is displaceably attached to the arm (20).

5. Device of anyone of claims 1 through 4, **characterized in that** the arcuate section (26, 80) has an angular scale (62, 96).

6. Device of anyone of claims 1 through 5, **characterized in that** the arm (20, 74) is formed as a U-shaped bow, the one leg of which forms the rod-shaped body (12, 72).

7. Device of claim 6, **characterized in that** the arcuate portion (26) is attached to the U-shaped body lying opposite the first end (14) of the rod-shaped body (12).

## Revendications

1. Dispositif pour guider un outil de perçage (56, 88) destiné à réaliser dans un os un perçage (58, 89, 95), qui coupe un premier perçage (54, 94, 101) déjà existant, comportant un premier corps (12, 72) en forme de tige, dont la première extrémité (14) est destinée à être introduite dans le premier perçage (54, 94, 101) déjà existant dans l'os et dont la deuxième extrémité (18) est munie d'un bras (20, 74) qui porte un guide (28, 82) pour l'outil de perçage (56, 88) dont l'axe longitudinal (33, 33') coupe le corps (12, 72) en forme de tige dans la zone de sa première extrémité (14), le bras (20, 74) comportant une partie (26, 80) en arc de cercle, le centre (36) de la partie (26, 80) en arc de cercle étant situé au point d'intersection de l'axe longitudinal (33, 33') de l'outil de perçage (56, 88) avec le corps (12, 72) en forme de tige, un orifice (16, 86) débouchant étant ménagé à travers le corps (12, 72) en forme de tige dans la zone du point d'intersection dans ce dernier, à travers lequel orifice l'outil de perçage (56, 56', 56") peut être guidé à travers le corps (12, 72) en forme de tige, dans lequel dispositif l'orifice (16, 86) est réalisé sous forme de forure débouchante, à travers laquelle l'outil de perçage (56, 56', 56") peut être guidé dans différentes positions angulaires, l'orifice (16) comporte des chanfreins (60), qui forment des guides d'introduction pour une pointe de l'outil de perçage (56, 56', 56"), et le guide (28, 82) de l'outil de perçage (56, 88) peut être déplacé le long de la partie (26, 80) en arc de cercle.

2. Dispositif selon la revendication 1, **caractérisé en ce que** la partie (26) en arc de cercle est montée de manière mobile sur le bras (20).

3. Dispositif selon la revendication 2, **caractérisé en ce que** la partie (26) en arc de cercle est montée de manière pivotante sur le bras (20).

4. Dispositif selon la revendication 2 ou 3, **caractérisé en ce que** la partie (26) en arc de cercle est montée de manière coulissante sur le bras (20).

5. Dispositif selon l'une quelconque des revendications 1 à 4, **caractérisé en ce que** la partie (26, 80) en arc de cercle est munie d'un rapporteur (62, 96).

6. Dispositif selon l'une quelconque des revendications 1 à 5, **caractérisé en ce que** le bras (20, 74) est réalisé sous forme d'arceau en U, dont une branche forme le corps (12, 72) en forme de tige.

7. Dispositif selon la revendication 6, **caractérisé en ce que** la partie (26) en arc de cercle est montée sur le corps en forme de U en face de la première extrémité (14) du corps (12) en forme de tige.
